# EUROPÄISCHE PATENTSCHRIFT

(11) **EP 0 777 740 B1**
(45) Veröffentlichungstag und Bekanntmachung des Hinweises auf die Patenterteilung: **05.04.2000**
(21) Anmeldenummer: 95931933.6
(22) Anmeldetag: 25.08.1995
(51) Int. Cl.: C12N 15/85, A61K 48/00, C07K 14/475, C12N 15/86, C12N 15/88

(54) **GENTHERAPEUTISCHE BEHANDLUNG VON ERKRANKUNGEN DES ZENTRALEN NERVENSYSTEMS (ZNS) DURCH EINEN ZELLSPEZIFISCHEN, ZELLZYKLUSABHÄNGIGEN WIRKSTOFF**
GENETIC THERAPY OF DISEASES OF THE CENTRAL NERVOUS SYSTEM WITH A CELL-SPECIFIC ACTIVE SUBSTANCE WHICH IS DEPENDENT ON THE CELL CYCLE
THERAPIE GENETIQUE DE MALADIES DU SYSTEME NERVEUX CENTRAL AVEC UNE SUBSTANCE ACTIVE SPECIFIQUE DE LA CELLULE ET DEPENDANT DU CYCLE CELLULAIRE

(30) Priorität: 26.08.1994 GB 9417366; 29.03.1995 GB 9506466
(43) Veröffentlichungstag der Anmeldung: 11.06.1997
(73) Patentinhaber: HOECHST AKTIENGESELLSCHAFT, 65926 Frankfurt am Main (DE)
(72) Erfinder: SEDLACEK, Hans-Harald, D-35041 Marburg (DE); MÜLLER, Rolf, D-35037 Marburg (DE)
(74) Vertreter: Dost, Wolfgang, Dr.rer.nat., Dipl.-Chem.
(86) Internationale Anmeldenummer: EP9503369
(87) Internationale Veröffentlichungsnummer: WO9606939

(56) Entgegenhaltungen:
- WO-A-94/04137
- WO-A-94/08026
- BRITISH JOURNAL OF HAEMATOLOGY, Bd. 87, Nr. 1(supplement), Juni 1994 Seite 124 F.EHLERT ET AL. 'Cell cycle-regulated transcription of the human cdc25C gene is controlled by a novel regulatory element'

## Beschreibung

### Technisches Gebiet

Es wird eine DNA-Sequenz für die Gentherapie von Erkrankungen des zentralen Nervensystems beschrieben

Die wesentlichen Elemente dieser DNA-Sequenz sind die Aktivatorsequenz, das Promotormodul und das Gen für die Wirksubstanz.

Die Aktivatorsequenz wird zellsppezifisch aktiviert in aktivierten Endothelzellen oder Gliazellen. Diese Aktivierung wird zellzyklusspezifisch reguliert durch das Promotormodul. Die Wirksubstanz stellt einen Nervenwachstumsfaktor, ein Enzym des Dopaninstoffwechsels und/oder einen Schutzfaktor für Nervenzellen dar. Die beschriebene DNA-Sequenz wird eingefügt in einen viralen oder nicht-viralen Vektor, ergänzt um einen Liganden mit Affinität für die Zielzelle.

### 1. Das zentrale Nervensystem und Wachstumsfaktoren

Nach Abschluß der Ontogenese stellen die Nervenzellen ausdifferenzierte, nicht mehr teilungsfähige Zellen dar. Sie sind im allgemeinen charakterisiert durch den Nervenzellkörper und Nervenzellfortsätze, wobei zuleitende (Dentrite) und ableitende (Neurite) Fortsätze unterschieden werden. Der pro Nervenzelle im allgemeinen einfach ausgebildete ableitende Neurit tritt mit seinem Zielorgen (Nerven- oder andersartige somatische Zellen) über Synapsen in Kontakt.

Die Aufrechterhaltung der anatomischen Struktur und der Funktion von Nervenzellen erfolgt unter der Anwesenheit von neuronalen Wachstumsfaktoren.

Unter neuronalen Wachstumsfaktoren sind im allgemeinen Sinne neurotrophe Faktoren zu verstehen (Übersichten bei Massague, Cell 49, 437 (1987), Pusztai et al., J. Pathol. 169, 191 (1993), Ibanez et al., PNAS 89, 3060 (1992), Sonoda et al., BBRC 185, 103 (1992)). Hierzu zählen neuronale Wachstumsfaktoren der Tabelle 1.

Im engeren Sinne sind hierzu zu zählen die Familie der Nervenwachstumsfaktoren (Nerve growth factors = NGF).

NGF wirken über die Bindung an NGF-Rezeptoren, die besonders an den sensorischen Nervenfasern ausgebildet sind. NGF wird intrazellulär aufgenommen und retrograd zum Nervenzellkörper transportiert (Johnson et al., J. Neurosci. 7, 923 (1987)). Dort bewirkt NGF wahrscheinlich einen Anstieg des zyklischen Adenosinmonophosphates (cAMP) mit nachfolgend erhöhtem Ca⁺⁺ Ausfluß (Schubert et al., Nature 273, 718 (1978) des weiteren über den Metabolismus von Inositollipiden die Freisetzung von Diacylglycerol und die Aktivierung von Proteinkinase C und über die Freisetzung von Inositoltriphosphat eine intrazelluläre Freisetzung von Ca⁺⁺ (Abdel-Latif, Pharmacol. Rev. 38, 227 (1986)).

Die hieraus resultierende Phosphorylierung von spezifischen, insbesondere von signalübertragenden Proteinen führt zu deren Funktionsänderungen. Ergebnis ist die verstärkte Bildung von Proteinen, die an dem Wachstum von Neuriten beteiligt sind. Hierzu gehören Chartin Proteine (Black et al., J. Cell Biol. 103, 545 (1986)), Tau Proteine und Tubuline (Drubin et al., J. Cell Biol. 101, 1799 (1985)). So ist die Synthese von α- und β-Tubulinen und von Neurofilamentproteinen (NF-L, NF-M, NF-H) und Peripherin (Portier et al., Devi Neuroscience 6, 215 (1983)), Parysek et al., J. Neurosci. 7, 781 (1987)) erhöht. Zugleich steigt die Konzentration von im Nervensystem wichtigen Enzymen an, wie beispielsweise Cholinacetyltransferase, Acetylcholinesterase und die Neuron-spezifische Enolase (Vinores et al., J. Neurochemistry 37, 597, 1981), Rydel et al., J. Neurosci. 7, 3639 (1987)).

Des weiteren werden die Konzentrationen von Neurotransmittern erhöht, wie beispielsweise Neurotensin (Tischler et al., Reg. Pept. 3, 415 (1982)) und Neuropeptid Y (Allen et al., Neurosci. Lett. 46, 291 (1984)) und von Neurotransmitterrezeptoren, wie beispielsweise Acetylcholinrezeptoren (Mitsuka et al., Brain Res. 314, 255 (1984)) und Enzephalinrezeptoren (Inoue et al., J. Biol. Chem. 257, 9238 (1982)). Zugleich ist die Konzentration von Synapsin I erhöht (Romano et al., J. Neurosci. 7, 1300 (1987)).

Im Endeffekt wird durch NGF der Funktionszustand von Nervenzellen aufrechterhalten. Zugleich initiiert und fördert NGF das Wachstum von Neuriten. Für diese neuritogene und synaptogene Wirksamkeit ist die andauernde Anwesenheit von NGF notwendig (Smith, Science 242, 708 (1988), Mitchison et al., Neuron 1, 761 (1988)).

Im besonderen konnte dies für Ciliary Neurotrophic Factor (CNTF) berichtet werden (Lin et al., Drugs of the Future 19, 557 (1994)).

Die neurotrophe Wirksamkeit von neuronalen Wachstumsfaktoren konnte experimentell, insbesondere bei Schädigungen von Nervenzellen, beispielsweise bei der chirurgischen Trennung von Neuriten, belegt werden. Wird CNTF lokal an den proximalen Stumpf der durchschnittenen Nerven verabreicht, wird der Anteil der nach dem chirurgischen Eingriff absterbenden Nervenzellen deutlich reduziert (Sendtner et al., Nature 345, 440 (1990)). Zugleich ist nach CNTF-Gabe die Konzentration, beispielsweise des Neuropeptids Substanz P, in den Spinalganglien deutlich erhöht. Ratten, denen der Ischiasnerv geschädigt wurde, zeigen nach subkutaner Applikation von CNTF eine beschleunigte Wiederherstellung ihrer motorischen Aktiviät (Lin et al., Drugs of the Future 19, 557 (1994)).

Die systemische Gabe von neurotrophischen Faktoren ist jedoch nur dann wirksam, wenn die im Rückenmark befindlichen, durch die Blut-Hirn-Schranke geschützten motorischen Neuronen noch funktionsfähige Axone außerhalb dieser Schranken besitzen, über welche die neurotrophen Faktoren aufgenommen werden können (Apfel et al., Brain Res. 605 1 (1993)).

Bei Nervenzellschädigungen bis jenseits oder jenseits der Blut-Hirn-Schranke ist die intracraniale Applikation von neurotrophen Faktoren notwendig. Hierdurch kann beispielsweise experimentell nach Durchtrennung der thalamischen Axone die retrograde Degeneration der proximalen thalamischen Neurone verhindert werden (Clatterbuch et al., PNAS 90, 2222 (1993)). Voraussetzung für einen optimalen Regenerationsprozess ist jedoch die dauernde Präsenz der neurotrophen Faktoren am Ort der geschädigten Nervenzelle. Die lokale Applikation ist zum Zeitpunkt der chirurgischen Schädigung oder Schadensbehebung möglich, jedoch nur schwierig oder fast unmöglich nach Abschluß des chirurgischen Eingriffs. Diffuse Schädigungen des ZNS, beispielsweise durch stumpfe Traumen oder Toxine, bieten nur im geringen Maße die Möglichkeit einer gezielten Applikation.

Durch traumatische, immunologische und toxische Einflüsse können Gliazellen angeregt werden, TNFα zu produzieren. Dieses TNFα ist seinerzeit toxisch für Nervenzellen und für Gliazellen (Owens et al., Immunol. Today 15, 566 (1994)).

Um eine möglichst langfristige Anwesenheit von Wirkstoffen im ZNS zu erzielen, wird versucht, Zellen intracranial (Fibroblasten, Endothelzellen, Myoblasten) zu injizieren, die in vitro transduziert wurden, neurotrophe Wirkstoffe zu exprimieren. Ziel ist, durch die neurotrophen Wirkstoffe die Regeneration und Funktion von traumatisch oder degenerativ geschädigten Nervenzellen, wie beispielsweise bei der Parkinsonschen Erkrankung oder bei der Dementia, zu verbessern. Speziell bei Parkinson wird versucht, Zellen zu injizieren, die entweder in vitro transduziert worden waren, die neurospezifischen Enzyme, wie Tyrosinhydroxylase und Dopadecarboxylase zu sekretieren (Kopin, Ann. Rev. Pharmacol. Toxicol. 32, 467 (1993), Fisher et al., Physiol. Rev. 11, 582 (1993), Jiao et al., Nature 362, 450 (1993)), oder aber es werden humane fetale, dopaminerge, nigrale Neuronen injziert (Löwenstein, Bio/Technology 12, 1075 (1994)).

Derartige Zellen sind jedoch nur begrenzt verfügbar. Zum anderen werden durch die Verwendung von fetalen Zellen erhebliche ethische Fragen aufgeworfen.

Als Alternative wird die Möglichkeit geprüft, Vektoren direkt in das Hirn zu injizieren, um Hirnzellen zu transduzieren, die gewünschten Wirkstoffe zu exprimieren (During et al., Science 266, 1399 (1994)). Da diese Vektoren jedoch keine Zellspezifität aufweisen, ist die erhebliche Gefahr gegeben, daß Nervenzellen durch die Infektion oder Transfektion mit dem Vektor Schäden erleiden können.

### 2. Beschreibung der Erfindung

Gegenstand der vorliegenden Erfindung ist nunmehr ein Wirkstoff, welcher lokal wie auch systemisch Patienten als Arzneimittel gegeben werden kann und durch welchen am Ort der Nervenzellschädigung über einen längeren Zeitraum neurospezifische Faktoren produziert werden. Derartige neurospezifische Faktoren können neurotrophe Faktoren sein, welche Nervenzellen vor weiteren Schäden schützen und die Regeneration von Nervenzellen bewirken. Neurospezifische Faktoren können jedoch auch Enzyme wie Tyrosinhydroxylase und Dopadecarboxylase sein, welche verantwortlich sind für die Synthese von Dopamin aus Tyrosin. Des weiteren können neurospezifische Faktoren Substanzen sein, welche TNFa inhibieren oder neutralisieren.

Zentraler Bestandteil dieses Wirkstoffes ist ein DNA-Konstrukt, welches aus folgenden Elementen besteht: 
Aktivatorsequenz
(UAS)
zellzyklusreguliertes
Promotormodul
neurospezifischer
Faktor
 (DNA wird im gesamten Text dieser Anmeldung als gemeinsamer Begriff sowohl für eine komplementäre (cDNA) wie auch für eine genomische DNA-Sequenz benutzt).

### 2.1. Wahl der Aktivatorsequenz

Als Aktivatorsequenz (UAS = upstream activator sequence) ist eine Nukleotidsequenz (Promotor- oder Enhancersequenzen) zu verstehen, mit der Transkriptionsfaktoren, gebildet oder aktiv in Endothel- oder Gliazellen, interagieren.

Als Aktivatorsequenz kann der CMV-Enhancer oder CMV-Promotor (EP-B1-0 173 177), der SV40 Promotor oder jeder andere, dem Fachmann bekannte Promotor- oder Enhancersequenz verwendet werden.

Im Sinne dieser Erfindung zählen jedoch zu den bevorzugten Aktivatorsequenzen genregulatorische Sequenzen bzw. Elemente aus Genen, die für besonders in Endothelzellen oder Gliazellen gebildete Proteine kodieren.

### a) Aktivatorsequenzen, aktiviert in Endothelzellen

Einige dieser Proteine sind von Burrows et al. (Pharmac. Therp. 64, 155 (1994) und Plate et al. (Brain Pathol. 4, 207 (1994)) beschrieben worden. Im besonderen zählen zu diesen Endothelzell-spezifischen Proteinen beispielsweise:
- Hirn-spezifischer, endothelialer Glucose-1-Transporter
   Endothelzellen des Hirns zeichnen sich durch eine sehr starke Expression dieses Transporters aus, um den transendothelialen Transport von D-Glucose in das Hirn zu bewerkstelligen (Gerhart et al., J. Neurosci. Res. 22, 464 (1989)). Die Promotorsequenz wurde von Murakami et al. (J. Biol. Chem. 267, 9300 (1992)) beschrieben.
- Endoglin
   Endoglin scheint ein nicht signalübertragender Rezeptor des TGFβ zu sein (Gangos et al., J. Biol. Chem. 265, 8361 (1990), Moren et al., BBRC 189, 356 (1992), Cheifetz, J. Biol. Chem. 267, 19027 (1992)). In geringen Mengen kommt er auf normalem Endothel vor, ist jedoch verstärkt exprimiert auf proliferierendem Endothel (Westphal et al., J. Invest. Derm. 100, 27 (1993), Burrows et al., Pharmac. Ther. 64, 155 (1994)). Die Promotorsequenzen wurden von (Bellon et al., Eur. J. Immunol. 23, 2340 (1993), Ge et al., Gene 138, 201 (1994)) beschrieben.
- VEGF-Rezeptoren
   Zwei Rezeptoren werden unterschieden (Plate et al., Int. J. Cancer 59, 520 (1994)):
   * VEGF-Rezeptor-1 (flt-1) (de Vries et al., Science 255, 989 (1992)) enthält im zytoplasmischen Teil eine fms-ähnliche Tyrosinkinase und der
   * VEGF-Rezeptor-2 (flk-1, KDR) (Terman et al., BBRC 187, 1579 (1992)) enthält im zytoplasmischen Teil eine Tyrosinkinase.

Beide Rezeptoren sind fast ausschließlich auf endothelialen Zellen (Senger et al., Cancer Metast. Rev. 12, 303 (1993)) anzutreffen.
- andere endothelspezifische Rezeptortyrosinkinasen
   * til-1 oder til-2 (Partanen et al., Mol. Cell Biol. 12, 1698 (1992), Schnürch und Risau, Developm. 119, 957 (1993), Dumont et al., Oncogene 7, 1471 (1992))
   * B61-Rezeptor
      (Eck-Rezeptor) (Bartley et al., Nature 368, 558 (1994), Pandey et al., Science 268, 567 (1995), Van der Geer et al., Ann. Rev. Cell Biol. 10, 251 (1994))
- B61
   Das B61 Protein ist der Ligand für den B61 Rezeptor. (Holzman et al., J. Am. Soc. Nephrol. 4, 466 (1993), Bartley et al., Nature 368, 558 (1994))
- Endothelin, im speziellen
   * Endothelin B (Oreilly et al., J. Cardiovasc. Pharm. 22, 18 (1993), Benatti et al., J. Clin. Invest. 91, 1149 (1993), O'Reilly et al., BBRC 193, 834 (1993)). Die Promotorsequenz wurde von Yanasigawa et al., Nature 332, 411 (1988) und Benatti et al., J. Clin. Invest. 91, 1149 (1993) beschrieben.
   * Endothelin-1 (Yanasigawa et al., Nature 332, 411 (1988)). Die Promotorsequenz wurde von Wilson et al., Mol. Cel.. Biol. 10, 4854 (1990) beschrieben.
   * Endothelin-Rezeptoren, insbesondere der Endothelin B-Rezeptor (Webb et al., Mol. Pharmacol. 47, 730 (1995), Haendler et al., J. Cardiovasc. Pharm. 20, 1 (1992)).
- Mannose-6-Phosphat-Rezeptoren
   (Perales et al., Eur. J. Biochem. 226, 225 (1994), Dahms et al., Cell 50, 181 (1987)).
   Die Promotorensequenzen sind von Ludwig et al. (Gene 142, 311 (1994)), Oshima et al. (J. Biol. Chem. 263, 2553 (1988)) und Pohlmann et al. (PNAS USA 85, 5575 (1987)) beschrieben worden.
- von Willebrand Faktor
   Die Promotorsequenz wurde von Jahroudi und Lynch (Mol. Cell. Biol. 14, 999 (1994), Ferreira et al., Biochem. J. 293, 641 (1993) und Aird et al., PNAS USA 92, 4567 (1995)) beschrieben.
- IL-1α,IL-1β.
   IL-1 wird von aktivierten Endothelzellen produziert (Warner et al., J. Immunol. 139, 1911 (1987).
   Die Promotorensequenzen wurden von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Mori et al., Blood 84, 1688 (1994), Hiscott et al., Mol. Cell. Biol. 13, 6231 (1993) beschrieben.
- IL-1-Rezeptor
   Die Promotorsequenz wurde von Ye et al., PNAS USA 90, 2295 (1993) beschrieben.
- Vascular Cell Adhesion Molecule (VCAM-1)
   Die Expression von VCAM-1 in Endothelzellen wird aktiviert durch Lipopolysaccharide, TNF-α (Neish et al., Mol. Cell. Biol. 15, 2558 (1995)), IL-4 (lademarco et al., J. Clin. Invest. 95, 264 (1995)), IL-1 (Marni et al., J. Clin. Invest. 92, 1866 (1993)).
   Die Promotorsequenz des VCAM-1 wurde beschrieben von Neish et al., Mol. Cell. Biol. 15, 2558 (1995), Ahmad et al., J. Biol. Chem. 270, 8976 (1995), Neish et al., J. Exp. Med. 176, 1583 (1992), lademarco et al., J. Biol. Chem. 267, 16323 (1992) und Cybulsky et al., PNAS USA 88, 7859 (1991).
- synthetische Aktivatorsequenzen
   Als Alternative zu natürlichen endothelspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen ...TTATCT... ist (Lee et al., Biol. Chem. 266, 16188 (1991); Dorfmann et al., J. Biol. Chem. 267, 1279 (1992) und Wilson et al., Mol. Cell. Biol. 10, 4854 (1990)).

### b) Aktivatorsequenzen, aktiviert in Gliazellen

Als bevorzugte Aktivatorsequenz ist des weiteren eine Nukleotidsequenz (Rromotor- oder Enhancersequenz) zu verstehen, welche mit Transkriptionsfaktoren, im besonderen Maße gebildet oder aktiv in Gliazellen, interagieren.

Hierzu zählen im besonderen genregulatorische Sequenzen bzw. Elemente aus Genen, die beispielsweise für folgende, in Gliazellen nachweisbare Proteine kodieren:
* das Schwannzell-spezifische Protein Periaxin (Gillespie et al., Neuron 12, 497 (1994))
   Die Promotorsequenzen wurden von Gillespie et al. (Neuron 12, 497 (1994))) beschrieben.
* Glutaminsynthetase
   (Akimoto et al., Brain Res. 72, 9 (1993), Fressinaud et al., J. Cell Physiol. 149, 459 (1991)).
   Die Promotorsequenzen wurden von Chakrabarti et al. (Gene 153, 163 (1995)) und Bhandarie et al. (J. Biol. Chem. 266, 7784 (1991)) beschrieben.
* das Gliazell-spezifische Protein
   (Glial fibrillary acidic protein = GFAP) (Akimoto et al., Brain Res. 72, 9 (1993))
   Die Promotorsequenzen wurden von Kumanishi et ai. (Acta Neuropath. 83, 569 (1992)), Besuard et al. (J. Biol. Chem. 266, 18877 (1991)), Reeves et al. (PNAS USA 86, 5178 (1989)), Brenner et al. (Brain Res. 7, 277 (1990)) und Masood et al. (J. Neurochem. 61, 160 (1993)) beschrieben.
* das Gliazellprotein S100b
   (Shen et al., Mol. Brain Res. 21, 62 (1994))
   Die Promotorsequenz wurde von Zimmer et al. (Brain Res. Bulletin 37, 417 (1995)) beschrieben.
* IL-6 (CNTF)
   (Sparacio et al., J. Neuroimmunol. 39, 231 (1992))
   Die Promotorsequenzen wurden von Chernajoosky et al. (J. Cell. Biochem. Suppl. 0/13, 73 (1989)), Ray et al. (Mol. Cell Biol. 10, 5736 (1990)), Droogmans et al. (DNA Sequence 3, 115 (1992)), Mori et al. (Blood 84, 2904 (1994)), Liberman et al. (Mol. Cell. Biol. 10, 2327 (1990)) und Ishiki et al. (Mol. Cell. Biol. 10, 2757 (1990)) beschrieben.
* 5-HT-Rezeptoren
   (Whitaker-Azmitia et al., Synapse 14, 201 (1993))
   Die Promotorsequenzen wurden von Elliott et al. (Neurochem. Int. 25, 537 (1994)), Veldman et al. (Mol. Pharmac. 42, 439 (1992)), Adham et al. (PNAS USA 90, 408 (1993)), Mochizuki et al. (BBRC 185, 517 (1992)) und Jin et al. (J. Biol. Chem. 267, 5735 (1992)) beschrieben.
* TNFα
   (Perez et al., Cell 63, 251 (1990), Merrill et al., J. Immunol. 151, 2132 (1993)).
   Die Promotorsequenzen wurden von Takashiba et al. (Gene 131, 307 (1993)) und van der Ahe et al. (Nucl. Acids Res. 21, 5636 (1993)) beschrieben.
* IL-10
   (Owens et al., Immunol. Today 15, 566 (1994)).
   Die Promotorsequenzen wurden von Kim et al. (J. Immunol. 148, 3618 (1992)), Kube et al. (Cytokine 7, 1 (1995) und Platzer et al. (DNA-Sequence 4, 399 (1994)) beschrieben.
* Insulin-like Growth Factor Receptor I and II
   Die Promotorsequenzen wurden von Morgan et al. (Nature 329, 301 (1987)), Cooke et al. (BBRC 177, 1113 (1991)), Kim et al. (Mol. Endocrin. 5, 1964 (1991)), van Dijk et al. (Mol. Cell. Endocrin. 81, 81 (1991)), Raizis et al. (Biochem. J. 289, 133 (1993)) und Yu et al. (Nature 371, 714 (1994)) beschrieben.
   - VEGF
      VEGF wird in vaskularisiertem Gewebe, besonders unter hypoxischen Bedingungen, gebildet (Berse et al., Mol. Biol. Cell 3, 211 (1992), Finkenzeller et al., BBRC 208, 432 (1995), Tischer et al., BBRC 165, 1198 (1989), Leung et al., Science 246, 1306 (1989), Ferrara et al., Endoc. Rev. 13, 18 (1992)). Die genregulatorische Sequenz für das VEGF-Gen ist
      * die Promotorsequenz des VEGF (5' flankierende Region)
         Michenko et al., Cell Mol. Biol. Res. 40, 35 (1994), Tischer et al., J. Biol. Chem. 266, 11947 (1991)) oder
      * die Enhancersequenz des VEGF-Gens (3' flankierende Region) (Michenko et al., Cell Mol. Biol. Res. 40, 35 (1994)) oder
      * das c-Src-Gen
         (Mukhopadhyay et al., Nature 375, 577 (1995), Bonham et al., Oncogene 8, 1973 (1993), Parker et al., Mol. Cell. Biol. 5, 831 (1985), Anderson et al., Mol. Cell. Biol. 5, 1122 (1985)) oder
      * das v-Src-Gen
         (Mukhodpadhyay et al., Nature 375, 577 (1995), Anderson et al., Mol. Cell. Biol. 5, 1122 (1985), Gibbs et al., J. Virol. 53, 19 (1985))

### 2.2. Wahl des Promotormoduls

Als zellzyklusreguliertes Promotormodul ist beispielsweise die Nukleotidsequenz -CDE-CHR-Inr- zu verstehen. Die wesentliche Funktion des Promotormoduls ist die Hemmung der Funktion der Aktivatorsequenz in der G0/G1 Phase des Zellzyklus und eine Zellzyklus-spezifische Expression in der S/G2 Phase und damit in proliferierenden Zellen.

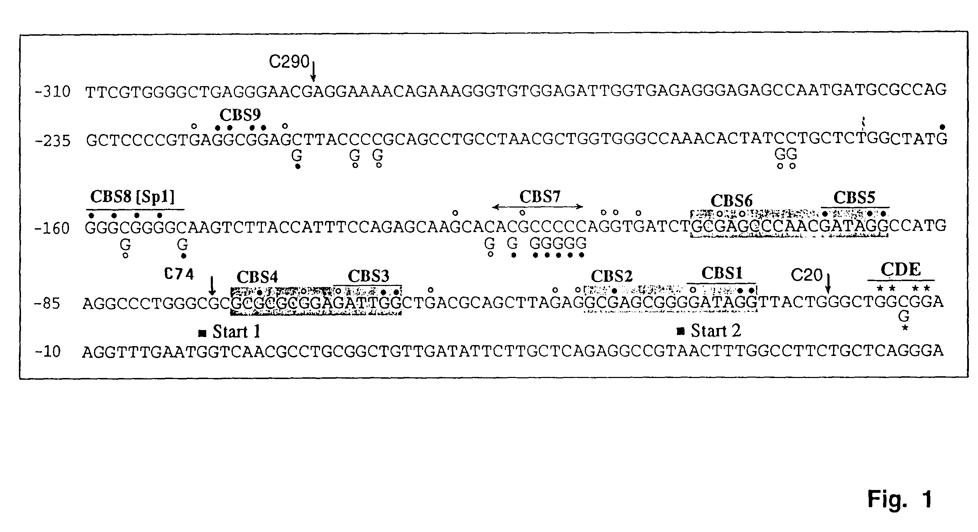

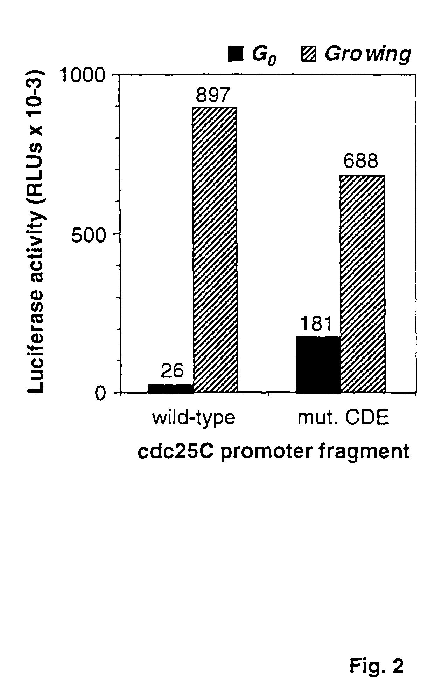

Das Promotormodul CDE-CHR-Inr wurde im Rahmen einer detaillierten Untersuchung der G2-spezifischen Expression des menschlichen cdc25C Promotors entdeckt. Ausgangspunkt war die Auffindung eines regulierenden Elementes ("cell cycle dependent element"; CDE), das für die Abschaltung des Promotors in der G1-Phase des Zellzyklus verantwortlich ist (Lucibello et al., EMBO J. 14, 132 (1995)). Durch genomisches Dimethylsulfat (DMS)-Footprinting und funktionelle Analysen (Fig. 1, 2) konnte gezeigt werden, daß das CDE einen Repressor ("CDE-binding factor"; CDF) G1-spezifisch bindet und hierdurch zu einer Inhibition der Transkription in nicht-proliferierenden (G0) Zellen führt. Das im Bereich des Basalpromotors lokalisierte CDE ist in seiner reprimierenden Funktion abhängig von einer "upstream activating sequence" (UAS).

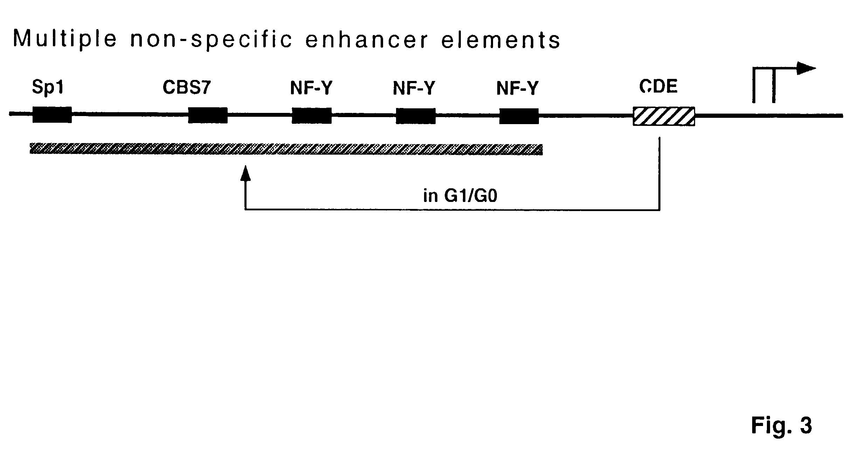

Dies führte zu dem Schluß, daß der CDE-bindende Faktor die transkriptionsaktivierende Wirkung 5' gebundener Aktivatorproteine in einer zellzyklusabhängigen Weise, d.h. in nicht-proliferierenden Zellen sowie in der G1-Phase des Zellzyklus, hemmt (Fig. 3).

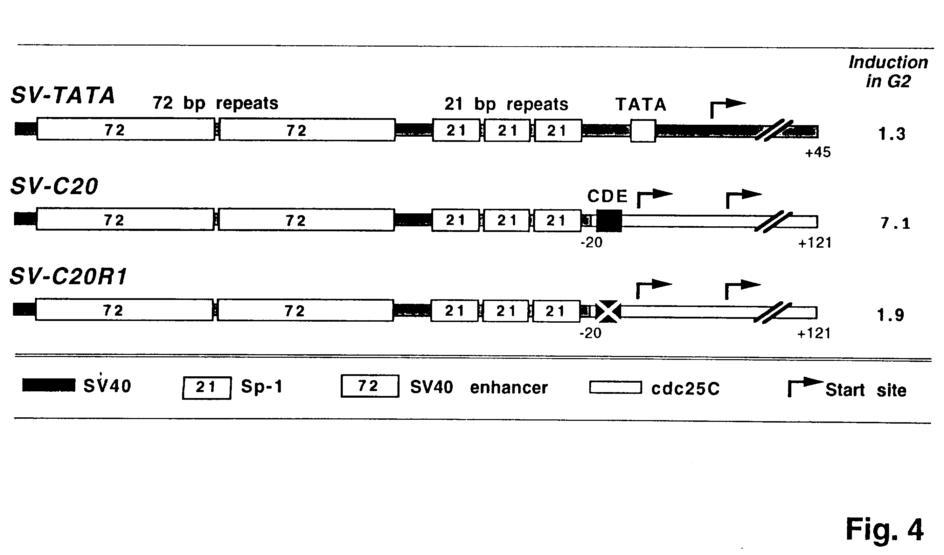

Diese Schlußfolgerung konnte durch ein weiteres Experiment bestätigt werden: Die Fusion des viralen, nicht-zellzyklusregulierten frühen SV40-Enhancers mit einem cdc25 Minimalpromotor (bestehend aus CDE und den 3' gelegenen Startstellen) führte zu einer klaren Zellzyklusregulation des chimären Promotors (Fig. 4). Nachfolgende Untersuchungen des cdc25C Enhancers haben gezeigt, daß es sich bei den vom CDF zellzyklusabhängig regulierten Transkriptionsfaktoren um NF-Y (CBF) (Dorn et al., Cell 50, 863 (1987), van Hujisduijnen et al., EMBO J. 9, 3119 (1990), Coustry et al., J. Biol. Chem. 270, 468 (1995)), Sp1 (Kadonaga et al., TIBS 11, 10 (1986)) und einen möglicherweise neuen an CBS7 bindenden Transkriptionsfaktor (CIF) handelt. Ein weiterer interessanter Befund dieser Studie war die Beobachtung, daß NF-Y innerhalb des cdc25C Enhancers nur in Kooperation mit mindestens einem weiteren NF-Y Komplex oder mit CIF effizient die Transkription aktiviert. Sowohl NF-Y als auch Sp1 gehören zur Klasse der glutaminreichen Aktivatoren, was wichtige Hinweise auf den Mechanismus der Repression (z.B. Interaktion bzw. Interferenz mit bestimmten basalen Transkriptionsfaktoren oder TAFs) liefert.

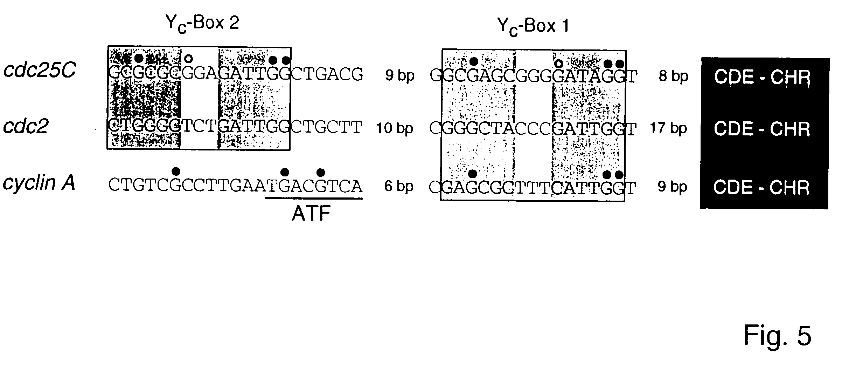

Ein Vergleich der Promotorsequenzen von cdc25C, cyclin A und cdc2 zeigte Homologien in mehreren Bereichen (Fig. 5). Nicht nur das CDE ist in allen 3 Promotoren konserviert (die vorhandenen Abweichungen sind funktionell nicht relevant), sondern auch die benachbarten Yc-Boxen. Alle diese Bereiche zeigten wie erwartet Proteinbindung in vivo, im Falle des CDE in zellzyklusabhängiger Weise. Außerdem konnte gezeigt werden, daß alle 3 Promotoren durch eine Mutation des CDE dereguliert werden (Tabelle 2). Eine bemerkenswerte Ähnlichkeit wurde bei dem Vergleich der cdc25C, cyclin A und cdc2 Sequenzen auch im Bereich unmittelbar 3' vom CDE ("cell cycle genes homology region"; CHR) deutlich (Fig. 5). Dieser Bereich ist funktionell ebenso bedeutsam wie das CDE (Tabelle 2), wird in den in vivo DMS Footprinting Experimenten jedoch nicht sichtbar.

Eine mögliche Erklärung hierfür ist eine Interaktion des Faktors mit der kleinen Furche der DNA. Ergebnisse aus "electrophoretic mobility shift assay" (EMSA) Experimenten deuten darauf hin, daß CDE und CHR gemeinsam einen Protein-komplex, den CDF, binden. Diese Beobachtungen legen den Schluß nahe, daß die CDF-vermittelte Repression glutaminreicher Aktivatoren ein häufig vorkommender Mechanismus zellzyklusregulierter Transkription ist.

Von Bedeutung für die Regulation des cdc25C Promotors ist jedoch anscheinend nicht nur der CDE-CHR-Bereich, sondern auch eine der Initiationsstellen (Position +1) innerhalb der Nukleotidsequenz des basalen Promotors (Positionen ≤ -20 bis ≥ +30, siehe Fig. 1). Mutationen in diesem Bereich, welcher die in vitro Bindestelle für den Transkriptionsfaktor YY-1 (Seto et al., Nature 354, 241 (1991), Usheva und Shenk Cell 76, 1115 (1994)), einschließt, führen zu einer völligen Deregulation. In Anbetracht der Nähe des CDE-CHR zum basalen Promotor ist somit eine Interaktion des CDF mit dem basalen Transkriptionskomplex sehr wahrscheinlich.

### 2.3. Wahl des neurospezifischen Faktors

a) neuronale Wachstumsfaktoren
   Als neurospezifischer Faktor im Sinne der Erfindung ist eine DNA-Sequenz zu verstehen, welche für einen neuronalen Wachstumsfaktor kodiert. Hierzu zählen beispielhaft insbesondere:
   - FGF
      (Johnson et al., Adv. Cancer Rec. 60, 1 (1993), Jay et al., Science 233, 541 (1986), Abrahahm et al., EMBO J. 5, 2523 (1986), Science 233, 545 (1986), Mergia et al., BBRC 138, 644 (1986), Schweigerer, Nature 325, 257 (1987), PNAS USA 84, 842 (1987))
   - Nerve growth factor (NGF)
      (Haktzopoulous et al., Neuron 13, 187 (1994), Takeda et al., Neuroscience 55, 23 (1993), Cartwright et al., Brain Res. 15, 67 (1992))
   - Brain-derived neurotrophic factor (BDNF)
      (Zhang et al., J. Neurobiol. 25, 1517 (1994), Maisonpierre et al., Genomics 10, 558 (1991), DNA Sequence 3, 49 (1992), Timmusk et al., Neuron 10, 475 (1993)).
   - Neurotrophin-3 (NT-3)
      (Hallboeoek et al., Eur. J. Neurosci. 5, 1 (1993), Rodriguez-Tebar et al., Philiosoph. Transact. Roy. Soc. Biol Sci. 331, 255 (1991), Leingärtner et al., Eur. J. Neurosci. 6, 1149 (1994))
   - Neurotrophin-4 (NT-4)
      (Ibanez et al., PNAS 89, 3060 (1992), Ny et al., PNAS 89, 3060 (1992))
   - Ciliary neurotrophic factor (CNTF)
      (Ishiki et al., New Biologist 3, 63 (1991), Ray et al., Mol. Cell Biol. 9, 5537 (1989), Leung et al., Neuron 8/6, 1045 (1992), Bootha et al., Gene 146, 303 (1994)).
b) Enzyme
   Als neurospezifischer Faktor ist des weiteren eine cDNA-Sequenz zu verstehen, welche kodiert für:
   - Tyrosinhydroxylase
      (Goc et al., Mol Cell Neurosci. 3, 383 (1992), Boularand et al., J. Biol. Chemistry 270, 3748 (1995)) oder für
   - Dopadecarboxylase
      (Maras et al., Eur. J. Biochem. 201, 385 (1991), Nayatsu, Neurosci. Res. 12, 315 (1991), Ichinose et al., Biochem. 31, 11546 (1992), Levanthai et al., Mol. Brain Res. 17, 227 (1993), Sumiichinose et al., J. Neurochem. 64, 514 (1995)).
c) Cytokine und deren Inhibitoren
   Als neurospezifischer Faktor ist des weiteren eine DNA-Sequenz zu verstehen, welche Proteine kodiert, die die neurotoxische Wirkung von TNFa inhibieren oder neutralisieren. Hierzu zählen beispielsweise:
   - TGFβ
      (Massague, Ann. Rev. Cell. Biol. 6, 597 (1990), Kondiah et al. J. Biol. Chem. 265, 1089 (1990), Garnier et al., J. Mol. Biol. 120, 97 (1978)). TGFβ inhibiert die TNFα-mediierte Zytotoxizität (Merrill et al., J. Immunol. 151, 2132 (1993), Quin et al., Annals of Surgery 220, 508 (1994))
   - lösliche TNF-Rezeptoren
      (Nophar et al., EMBO J. 9, 3269 (1990), Himmler et al., DNA Cell Biol. 9, 705 (1990), Aggarwal et al., Nature 318, 665 (1985), Gray et al., PNAS 87, 7380 (1990), Tartaglia et al., Immunol. Today 13, 151 (1992), Loetcher et al., Cell 61, 351 (1990), Schall et al., Cell 61, 361 (1990), Smith et al., Science 248, 1019 (1990), Goodwin et al., Mol. Cell. Biol. 11, 3020 (1991)).
      TNF-Rezeptoren neutralisieren TNFα.Übersicht: Olsson et al., Eur. Cytokine Netw. 4, 169 (1993).
   - IL-10
      (Moore et al., Science 248, 1230 (1990), Vieira et al., PNAS USA 88, 1172 (1991), Kim et al., J. Immunol. 148, 3618 (1992)).
      IL-10 inhibiert die Bildung von IFN gamma, TNFα, IL-2 und IL-4 (Schlaak et al., Scand. J. Immunol. 39, 209 (1994), Vieira et al., PNAS USA 88, 1172 (1991), Benjamin et al., Leuk. Lymph. 12, 205 (1994))
   - lösliche IL-1-Rezeptoren
      * IL-1-Rezeptor I
         (Sims et al., PNAS USA 86, 8946 (1989), Dower et al., J. Exp. Med. 162, 501 (1985), Chizzonite et al., PNAS 86, 8029 (1989)
      * IL-1-Rezeptor II
         (McMahan et al., EMBO J. 10, 2821 (1991), Sims et al., Science 241, 585 (1988)).
         Lösliche IL-1-Rezeptoren neutralisieren die Aktivität von IL-1 (Colotta et al., Immunol. Today 15, 562 (1994), Sims et al., Clin. Immunol. Immunopath. 72, 9 1994))
   - IL-1-Rezeptor-Antagonist
      (Eisenberg et al., Nature 343, 341 (1990), Carter et al., Nature 344 (633 (1990))
   - lösliche IL-6-Rezeptoren
      (Mackiewicz et al., Cytokine 7, 142 (1995))

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der Rezeptoren zum einen und dem Fc-Teil des menschlichen Immunglobulins zum anderen Verwendung finden. Derartige DNA-Sequenzen und ihre Herstellung wurden in der EP 0 464 633 A1 beschrieben.

### 2.4. Kombination von mehreren neurospezifischen Faktoren

Gegenstand der Erfindung ist des weiteren ein Wirkstoff, in welchem eine Kombination der DNA-Sequenzen von gleichen neurospezifischen Faktoren (A,A) oder von unterschiedlichen neurospezifischen Faktoren (A,B) vorliegt. Zur Expression zweier DNA-Sequenzen ist vorzugsweise die cDNA einer "internal ribosome entry site" (IRES) als regulatorisches Element zwischengeschaltet.

Aktivator-
sequenz
(UAS)

zellzyklus-
reguliertes
Promotor-
modul

neurospezi-
fischer
Faktor
A

internal
ribosome
entry
site

neurospezi-
fischer
Faktor
A oder B

Derartige IRES wurden beispielsweise von Montford und Smith (TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position _< 140 bis _> 630 des 5' UTR (Pelletier und Sonenberg, Nature 334, 320 (1988)) zur Verknüpfung der DNA der antientzündlichen Substanz A (am 3' Ende) und der DNA der antientzündlichen Substanz B (am 5' Terminus) verwendet werden.

Ein derartiger Wirkstoff weist je nach Kombination additive (A+A, A+B1) oder synergistische Wirkung im Sinne der Erfindung auf.

### 2.5. Konstruktion des Vektors

Das erfindungsgemäße DNA-Konstrukt wird in einer dem Fachmann geläufigen Weise zu einem Vektor vervollständigt. Dieser Vektor kann viralen oder nicht-viralen Ursprungs sein. Beispielsweise wird das erfindungsmäße DNA-Konstrukt in einen viralen Vektor eingefügt (siehe hierzu D. Jolly, Cancer Gene Therapy 1, 51 (1994)), oder aber zu einem Plasmid ergänzt. Virale Vektoren oder Plasmide können mit kolloidalen Dispersionen komplexiert werden. Hierzu zählen beispielsweise Liposomen (Farhood et al., Annals of the New York Academy of Sciences 716, 23 (1994)) oder aber Polylysin-Ligand-Konjugate (Curiel et al., Annals of the New York Academy of Sciences 716, 36 (1994)).

### 2.6. Auswahl des Liganden

Virale und nicht-virale Vektoren können mit einem Liganden ergänzt werden. Als Ligand, beispielsweise in Polylysin-Ligand-Konjugaten, werden Substanzen bevorzugt, welche an die Oberfläche von Endothelzellen binden. Hierzu gehören Antikörper oder Antikörperfragmente, gerichtet gegen Membranstrukturen von Endothelzellen wie sie beispielsweise von Burrows et al. (Pharmac. Ther. 64, 155 (1994)) oder in der EP 0 408 859 A2 beschrieben wurden. Insbesondere zählen hierzu Antikörper gegen die VEGF-Rezeptoren.

Die murinen monoklonalen Antikörper sind bevorzugt in humanisierter Form einzusetzen. Die Humanisierung erfolgt in der von Winter et al. (Nature 349, 293 (1991) und Hoogenboom et al. (Rev. Tr. Transfus. Hemobiol. 36, 19 (1993) dargestellten Weise. Antikörperfragmente werden gemäß dem Stand der Technik hergestellt, beispielsweise in der von Winter et al., Nature 349, 293 (1991), Hoogenboom et al., Rev. Tr. Transfus. Hemobiol. 36, 19 (1993), Givol, Mol. Immunol. 28, 1379 (1991) oder Huston et al., Int. Rev. Immunol. 10, 195 (1993) beschriebenen Weise.

Hierzu gehören des weiteren alle Wirkstoffe, welche an Membranstrukturen oder Membranrezeptoren auf Endothelzellen binden. Beispielsweise gehören hierzu Wachstumsfaktoren oder deren Fragmente bzw. Teilsequenzen von ihnen, die an Rezeptoren exprimiert durch Endothelzellen binden, wie beispielweise PDGF, bFGF, VEGF, TGFβ (Pusztal et al., J. Pathol. 169, 191 (1993)). Des weiteren zählen hierzu Substanzen, die endständig Mannose tragen und an den Mannose-6-Phosphatrezeptor von Endothelzellen binden (Perales et al., Eur. J. Biochem. 226, 225 (1994)).

Des weiteren gehören hierzu Adhäsionsmoleküle, welche an aktivierte und/oder proliferierende Endothelzellen binden. Derartige Adhäsionsmoleküle, wie beispielsweise SLeX, LFA-1, MAC-1, LECAM-1 oder VLA-4, wurden bereits beschrieben (Übersichten bei Augustin-Voss et al., J. Cell Biol. 119, 483 (1992), Pauli et al., Cancer Metast. Rev. 9, 175 (1990), Honn et al., Cancer Metast. Rev. 11, 353 (1992)).

Als Ligand sind des weiteren Substanzen anzusehen, welche an die Oberfläche von Gliazellen binden.

Hierzu gehören Antikörper oder Antikörperfragmente gerichtet gegen Membranstrukturen von Gliazellen, wie sie beispielsweise von Mirsky et al. (Cell and Tissue Res. 240, 723 (1985) von Coakham et al., (Prog. Exp. Tumor Rex. 29, 57 (1985)) und von McKeever et al. (Neurobiol. 6, 119

(1991)) berichtet wurden. Zu diesen Membranstrukturen gehören des weiteren Neuraladhäsionsmoleküle wie N-CAM, inbesondere dessen Polypeptidkette C (Nybroe et al., J. Cell Biol. 101, 2310 (1985)).

Hierzu gehören des weiteren alle Wirkstoffe, welche an Membranstrukturen oder Membranrezeptoren auf Gliazellen binden. Beispielsweise gehören hierzu Substanzen, die endständig Mannose tragen und an den Mannose-6-Phosphatrezeptor binden (Perales et al., Eur. J. Biochem. 226, 225 (1994), Insulin und Insulin-like growth factor (Merrill et al., J. Clin. Endocrin. Metab. 71, 199 (1990)), PDGF (Ek et al., Nature 295, 419 (1982)) und diejenigen Fragmente dieser Wachstumsfaktoren, welche an die zugehörigen Membranrezeptoren binden.

### 2.7. Herstellung des Wirkstoffes

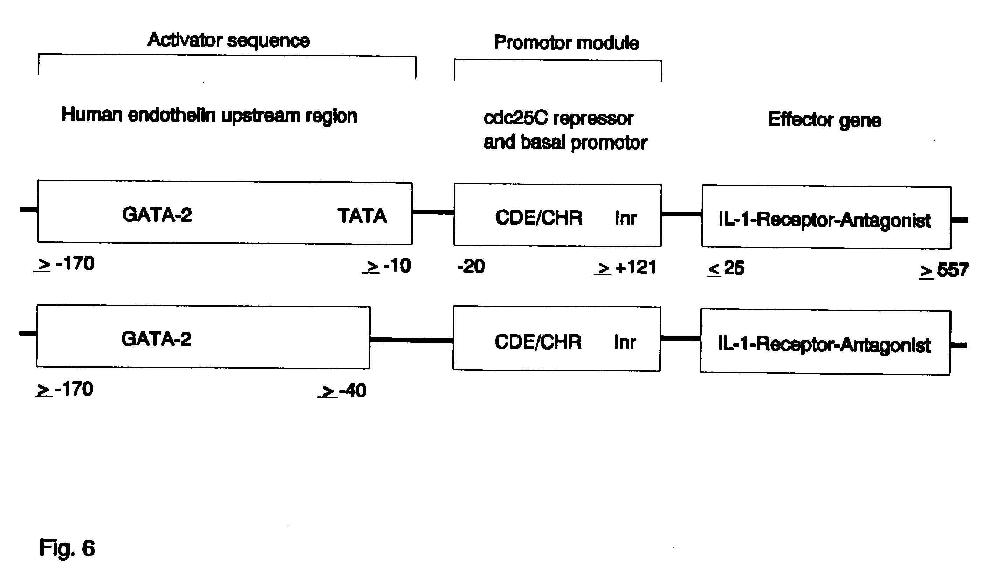

Herstellung des erfindungsgemäßen Wirkstoffes wird anhand folgenden Beispieles näher beschrieben:
a) Konstruktion des chimären Promotors Endothelin 1CDE-CHR-Inr
   Der humane Endothelin-1 Promotor (Position ≤ -170 bis ≥ -10) oder eine um die TATA-Box verkürzte Variante (Position ≤ -170 bis ≥ -40) werden an ihrem 3' Ende mit dem 5'-Terminus des CDE-CHR-Inr Moduls (Position ≤ -20 bis ≥ +121) des humanen cdc25C-Gens verknüpft (Fig. 6). Die Verknüpfung erfolgt mit Hilfe von dem Fachmann bekannten und käuflichen Enzymen.
b) Konstruktion eines Plasmids enthaltend den zentralen Bestandteil des Wirkstoffes
   Die so hergestellte chimäre Endothelin-1, Repressormodul-Transkriptionseinheit wird an ihren 3' Enden mit dem 5'-Terminus einer DNA, die den kompletten kodierenden Bereich des 152 Aminosäuren langen IL-1-Rezeptor-Antagonisten enthält (DNA Position ≤ 25 bis ≥ 557; Eisenberg et al., Nature 343, 341 (1990)), verknüpft. Diese DNA enthält auch die für eine Sekretion notwendige Signalsequenz (25 N-terminale Aminosäuren). Transkriptionskontrolleinheiten und IL-1-Rezeptor-Antagonist DNA werden in pUC19/19 oder Bluescript-abgeleiteten Plasmidvektoren einkloniert, die direkt (Yovandich et al., Hum. Gene Ther. 6, 603 (1995)) oder in kolloidalen Dispersionssystemen für einen in vivo Transfer genutzt werden können.
   Alternativ können die zusammengeführten Transkriptionskontrolleinheiten und IL-1-Rezeptor-Antagonisten DNA in dem Fachmann geläufige virale Vektoren oder andere nicht-virale Vektoren transferiert werden.

### 2.8. Wirksamkeit des Wirkstoffes

Ein Wirkstoff gemäß der vorliegenden Erfindung ermöglicht nach lokaler Applikation beispielsweise an den Ort der Nervenschädigung oder intrakranieller bzw. subarachnoidaler Gabe oder systemischer, bevorzugt intravenöser oder intraarterieller Gabe, daß durch den gewebespezifischen Enhancer und durch den basalen Promotor vorwiegend, wenn nicht ausschließlich nur proliferierende Endothelzellen oder proliferierende Gliazellen neurospezifische Faktoren ausscheiden. Derartige Endothelzellproliferationen oder Gliazellproliferationen sind im Bereich und als Reaktion auf Gewebeschädigung zu erwarten, welche zugleich auch die Nervenschädigung verursacht hat. Der erfindungsgemäße Wirkstoff gewährleistet somit eine hohe Konzentration des neurospezifischen Faktors am Ort der Nervenschädigung.

Da der Wirkstoff sowohl durch seine Zell- als auch Zellzyklusspezifität ein hohes Maß an Sicherheit verspricht, kann er auch in hohen Dosierungen und, falls notwendig, mehrmals in Abständen von Tagen oder Wochen zur Prophylaxe oder zur Therapie von Nervenschädigungen angewandt werden.

### Legende zu Fig. 1 - 6:

Fig. 1:
Nukleotidsequenz des cdc25C - Promotorbereichs mit den in vivo gefundenen Proteinbindungsstellen (genomisches DMS Footprinting; · (gefüllte Kreise): vollständiger konstitutiver Schutz; o (offene Kreise): partieller konstitutiver Schutz; * (Sternchen): zellzyklusregulierter, G1-spezifischer Schutz). CBS: constitutive binding site; CDE: cell cycle-dependent element. Grau unterlegte Bereiche zeigen die Y_{c}-Boxen (NF-Y Bindestellen) an. Startstellen sind durch gefüllte Quadrate markiert.
Fig. 2:
Derepression des cdc25C-Promotors spezifisch in G₀ durch Mutation des cdc.
Fig. 3:
Schematische Darstellung der cdc25C Enhancer-Regulation durch das CDE.
Fig. 4:
G₀ / G₁ - spezifische Repression des SV40-Enhancers durch das CDE.
Fig. 5:
Homologien im CDE-CHR-Bereich und den 5' gelegenen Yc-Boxen in den cdc25C, cyclin A und cdc2-Promotoren
Fig. 6:
Chimäre Konstrukte bestehend aus verschiedenen Anteilen des humanen Endothelin-1 Promotors, dem 3' fusionierten Promotormodul mit den CDE und CHR Repressorelementen sowie einer DNA für den IL-1-Rezeptor-Antagonisten (kompletter kodierender Bereich Position ≤ 25 bis ≥ 557; Eisenberg et al., Nature 343, 341 (1989)) als Effektor. Positionsangaben beziehen sich auf die Angaben von Wilson et al., Mol. Cell. Biol. 10, 4854 (1990) für das Endothelin-1 Gen bzw. auf das von Lucibello et al., EMBO J. 14, 132 (1995) verwendete System für cdc25C.

**Tabelle 1:**

| Neuronale Wachstumsfaktoren | | |
|---|---|---|
| | Bildungsort | Wirkort |
| Familie der "Epidermal growth factors" | | |
| | | |
| Schwannoma-derived growth factor (SDGF) | Schwannsche Zellen | Astrozyten Schwannsche Zellen Fibroblasten |
| | | |

| Familie der "Heparin binding growth factors" | | |
|---|---|---|
| | | |
| acidic-fibroblast growth factor (aFGF) | ubiquitär | ubiquitär |
| | | |
| basic-fibroblast growth factor (bFGF) | ubiquitär | ubiquitär |
| | | |

| Familie der "Nerve growth factors" | | |
|---|---|---|
| nerve growth factor (NGF) | Schwannsche Zellen Neuronen Melanozyten cholinergische Neuronen im Gehirn | periphere Neuronen |
| | | |
| brain derived neurotrophic factor (BDNF) | Neuronen Gliazellen | dopaminergische Neuronen im Gehirn |
| Neurotrophin-3, -4 (NT-3, NT-4) | viele Zelltypen | periphere propriozeptive Neuronen |
| | | |
| Ciliary neurotrophic factor (CNTF) | | periphere Nervenzellen |

**Tabelle 2:**

| Rolle von CDE und CHR bei der zellzyklusregulierten Transkription von cdc25C, cyclin A und cdc2 | | | |
|---|---|---|---|
| ***Tab. 2*** | | | |
| | ***G***_{***0***} | ***Growing*** | ***Factor*** |
| ***wt*** | | | |
| cdc25C | 0.8 | 13.1 | 17.5 |
| cyclin A | 0.7 | 27.1 | 41.7 |
| cdc2 | 1.0 | 41.2 | 41.2 |

| ***mCDE(-13)*** | | | |
|---|---|---|---|
| cdc25C | 7.6 | 11.6 | 1.5 |
| cyclin A | 13.4 | 23.9 | 1.8 |
| cdc2 | 11.3 | 33.9 | 3.0 |

| ***mCHR(-6/-3)*** | | | |
|---|---|---|---|
| cdc25C | 14.4 | 21.0 | 1.5 |
| cyclin A | 15.5 | 28.3 | 1.8 |
| cdc2 | 18.6 | 38.6 | 2.1 |
| Ergebnisse transienter Transfektionen in HIH3T3-Zellen sind als RLUs/1000 dargestellt. mCDE: mutiertes CDE (Pos. -13: Ġ → T); mCHR: mutiertes CHR (Pos. -6 bis -3). | | | |

## Patentansprüche

1. Wirkstoff zur Prophylaxe oder Therapie von Erkrankungen des zentralen Nervensystems, charakterisiert dadurch, daß er ein DNA-Konstrukt enthält, welches aus einer Aktivatorsequenz, einem zellzyklusregulierten Promotormodul und einer DNA-Sequenz für einen neurospezifischen Faktor besteht.

2. Wirkstoff nach Anspruch 1), bei welchem das Promotormodul die Elemente CDE-CHR-Inr besitzt und die Positionen ≤ -20 bis ≥ +30 des cdc25C Promotorbereiches enthält (Nukleotidsequenz (GCTGGCGGAAGGTTTGAATGGTCAACGCCTGCGGCTGTTGATATTCT TG), wobei CDE das "cell cycle dependent element" (Nukleotid- sequenz TGGCGG), CHR die "cell cycle gene homology region" (Nukleotidsequenz GTTTGAA) und Inr die Initiationsstelle (Position +1) sowie die für die Initiation wichtigen benachbarten Sequenzen darstellen, sowie funktionell aktive Varianten und Mutanten des Promotormoduls ebenfalls beinhaltet sind.

3. Wirkstoff nach Anspruch 1), enthaltend eine Aktivatorsequenz, welche durch Transkriptionsfaktoren, gebildet in Endothelzellen oder in Gliazellen, reguliert wird.

4. Wirkstoff nach Anspruch 2), enthaltend als Aktivatorsequenz
- den CMV-Promotor, den CMV-Enhancer oder den SV40 Promotor, oder
- die Promotorsequenz für den Hirn-spezifischen, endothelialen Glucose-1-Transporter, für Endoglin, für VEGF-Rezeptor,1 -2, die Rezeptortyrosinkinase til-1 oder til-2, den B61-Rezeptor, den B61-Liganden für Endothelin, im besonderen Endothelin B oder Endothelin-1, für Endothelin-Rezeptoren, für Mannose-6-Phosphatrezeptor, IL-1α oder IL-1β, IL-1-Rezeptor, VCAM-1, von Willebrand Faktor oder
- oligomerisierte Bindungsstellen für Transkriptionsfaktoren, die preferentiell oder selektiv in Endothelzellen aktiv sind, wie beispielsweise GATA-2 mit seiner Bindestelle 5'-TTATCT-3' oder
- Promotorsequenzen für das Schwannzell-spezifische Periaxin, für Glutaminsynthetase, das Glia-spezifische Protein, das Gliazellprotein S100b, Interleukin-6, 5-Hydroxytryptamin-Rezeptoren, TNFα, IL-10 oder Insulin-like Growth Factor Rezeptoren oder
- die Promotorsequenz von VEGF, die Enhancersequenz von VEGF oder die v-Src DNA-Sequenz oder die c-Src DNA-Sequenz, welche das VEGF-Gen regulieren.

5. Wirkstoff nach den Ansprüchen 1-3), bei welchem die DNA-Sequenz für den neurospezifischen Faktor
- einen neuronalen Wachstumsfaktor, im besonderen FGF, NGF, BDNF, -NT-3, NT-4 oder CNTF oder
- TGFβ, einen löslichen TNF-Rezeptor, IL-10, einen löslichen IL-1-Rezeptor oder einen löslichen IL-6-Rezeptor oder den IL-1-Rezeptorantagonisten oder ein Fusionsprotein von einem Cytokin (z.B. TGFβ, IL-10 oder IL-1-Rezeptorantagonist) oder einem löslichen Cytokin-Rezeptor (z.B. TNF-Rezeptor, IL-6-Rezeptor oder IL-1-Rezeptor) mit dem Fc-Teil des menschlichen Immunglobulins kodiert oder
- eine Tyrosinhydroxylase oder Dopadecarboxylase kodiert.

6. Wirkstoff nach Anspruch 1-5), welcher die DNA-Sequenzen von zwei gleichen oder zwei unterschiedlichen neurospezifischen Faktoren enthält, wobei die zwei DNA-Sequenzen durch eine DNA-Sequenz für die internal ribosome entry site miteinander verbunden sind.

7. Wirkstoff nach den Ansprüchen 1-6), eingefügt in einen Vektor.

8. Wirkstoff nach Anspruch 7), bei welchem der Vektor ein Virus ist.

9. Wirkstoff nach Anspruch 8), bei welchem das Virus ein Retrovirus, Adenovirus, Adeno-assoziiertes Virus, Herpes Simplex Virus oder Vaccinia Virus darstellt.

10. Wirkstoff nach Anspruch 1-6), eingefügt in ein Plasmid.

11. Wirkstoff nach Anspruch 7-10), zubereitet in einem kolloidalen Dispersionssystem.

12. Wirkstoff nach Anspruch 11), bei welchem das kolloidale Dispersionssystem Liposome sind.

13. Wirkstoff nach Anspruch 12), bei welchem das kolloidale Dispersionssystem Polylysin-Liganden sind.

14. Wirkstoff nach Anspruch 7-13), welcher um einen Liganden ergänzt ist, der an Membranstrukturen von Endothelzellen oder Gliazellen bindet.

15. Wirkstoff nach Anspruch 14), bei welchem der Ligand
- ein polyklonaler oder monoklonaler Antiköper oder ein Antikörperfragment hiervon ist, der mit seinen variablen Domänen an Membranstrukturen von Endothelzellen oder Gliazellen bindet, oder
- eine endständige Mannose-tragende Substanz, ein Cytokin oder Wachstumsfaktor oder ein Fragment bzw. eine Teilsequenz hiervon ist, die bzw. der an Rezeptoren auf Endothelzellen oder Gliazellen bindet.

16. Wirkstoff nach Anspruch 15), bei welchem die Membranstrukturen
- ein Rezeptor für ein Cytokin oder einen Wachstumsfaktor, wie IL-1, FGF, PDGF, VEGF (im besonderen FIT-1 und KDR), TGFβ, Insulin oder Insulin-like Growth Factor (ILGF) darstellt oder
- ein Adhäsionsmolekül darstellt, wie SLeX, LFA-1, MAC-1, LECAM-1 oder VLA-4
- oder der Mannose-6-Phosphat-Rezeptor darstellt.

17. Wirkstoff nach den Ansprüchen 1-16) in einer Arzneimittelzubereitung für die intravenöse oder intraarterielle Injektion, für die lokale Applikation am Ort eines Traumas oder für die Injektion in die Hohlräume des zentralen Nervensystems.

## Claims

1. An active compound for the prophylaxis or therapy of diseases of the central nervous system, which contains a DNA construct which is composed of an activator sequence, a cell cycle-regulated promoter module and a DNA sequence for a neurospecific factor.

2. The active compound as claimed in claim 1), wherein the promoter module possesses the elements CDE-CHR-Inr and contains positions ≤ -20 to ≥ +30 of the cdc25C promoter region (nucleotide sequence GCTGGCGGAAGGTTTGAATGGTCAACGCCTGCGGCTGTTGATATTCTTG), where CDE constitutes the cell cycle dependent element (nucleotide sequence TGGCGG), CHR constitutes the cell cycle gene homology region (nucleotide sequence GTTTGAA) and Inr constitutes the initiation site (position +1) and also the adjacent sequences which are important for initiation, and also functionally active variants and mutants of the promoter module are likewise included.

3. The active compound as claimed in claim 1), which contains an activator sequence which is regulated by transcription factors which are formed in endothelial cells or in glial cells.

4. The active compound as claimed in claim 2), which contains, as activator sequence,
- the CMV promoter, the CMV enhancer or the SV40 promoter, or
- the promoter sequence for the brain-specific, endothelial glucose-1 transporter, for endoglin, for VEGF receptor 1 and 2, the receptor tyrosine kinase til-1 or til-2, the B61 receptor, the B61 ligand for endothelin, in particular endothelin B or endothelin 1, for endothelin receptors, for mannose 6-phosphate receptor, IL-1α or IL-1β, IL-1 receptor, VCAM-1 or von Willebrand factor, or
- oligomerized binding sites for transcription factors which are preferentially or selectively active in endothelial cells, such as GATA-2 with its binding site 5'-TTATCT-3', or
- promoter sequences for Schwann cell-specific periaxin, for glutamine synthetase, the glia-specific protein, the glial cell protein S100b, interleukin-6, 5-hydroxytryptamine receptors, TNFa, IL-10 or insulin-like growth factor receptors, or
- the promoter sequence of VEGF, the enhancer sequence of VEGF or the v-Src DNA sequence or the c-Src DNA sequence, which regulate the VEGF gene.

5. The active compound as claimed in claims 1-3), wherein the DNA sequence for the neurospecific factor encodes
- a neuronal growth factor, in particular FGF, NGF, BDNF, NT-3, NT-4 or CNTF, or
- TGFβ, a soluble TNF receptor, IL-10, a soluble IL-1 receptor or a soluble IL-6 receptor or the IL-1 receptor antagonist or a fusion protein of a cytokine (e.g. TGFβ, IL-10 or IL-1 receptor antagonist) or a soluble cytokine receptor (e.g. TNF receptor, IL-6 receptor or IL-1 receptor) with the Fc moiety of human immunoglobulin, or
- encodes a tyrosine hydroxylase or dopa decarboxylase.

6. The active compound as claimed in claim 1-5), which contains the DNA sequences of two identical or two different neurospecific factors, with the two DNA sequences being connected to each other through a DNA sequence for the internal ribosome entry site.

7. The active compound as claimed in claims 1-6), which is inserted into a vector.

8. The active compound as claimed in claim 7), wherein the vector is a virus.

9. The active compound as claimed in claim 8), wherein the virus is a retrovirus, adenovirus, adeno-associated virus, herpes simplex virus or vaccinia virus.

10. The active compound as claimed in claim 1-6), which is inserted into a plasmid.

11. The active compound as claimed in claim 7-10), which is prepared in a colloidal dispersion system.

12. The active compound as claimed in claim 11), wherein the colloidal dispersion system are liposomes.

13. The active compound as claimed in claim 12), wherein the colloidal dispersion system are polylysine ligands.

14. The active compound as claimed in claim 7-13), which is supplemented by a ligand which binds to membrane structures of endothelial cells or glial cells.

15. The active compound as claimed in claim 14), wherein the ligand
- is a polyclonal or monoclonal antibody, or an antibody fragment thereof, which binds with its variable domains, to membrane structures of endothelial cells or glial cells, or
- is a substance which carries mannose terminally, a cytokine or growth factor or a fragment, or a part of a sequence thereof, which binds to receptors on endothelial cells or glial cells.

16. The active compound as claimed in claim 15), wherein the membrane structures
- constitutes a receptor for a cytokine or a growth factor, such as IL-1, FGF, PDGF, VEGF (in particular FIT-1 and KDR), TGFβ, insulin or insulin-like growth factor (ILGF), or
- constitutes an adhesion molecule, such as SLeX, LFA-1, MAC-1, LECAM-1 or VLA-4
- or constitutes the mannose 6-phosphate receptor.

17. The active compound as claimed in claims 1-16) in a pharmaceutical preparation for intravenous or intraarterial injection, for local administration at the site of a trauma or for injection into the cavities of the central nervous system.

## Revendications

1. Substance active pour la prophylaxie ou la thérapie de maladies du système nerveux central, caractérisée en ce qu'elle contient un produit de construction de type ADN qui est constitué d'une séquence d'activateur, d'un module promoteur régulé par le cycle cellulaire et d'une séquence d'ADN codant pour un facteur neurospécifique.

2. Substance active selon la revendication 1, dans laquelle le module promoteur possède les éléments CDE-CHR-Inr et contient les positions ≤ -20 à ≥ +30 de la région de promoteur cdc25C (séquence nucléotidique GCTGGCGGAAGGTTTGAATGGTCAACGCCTGCGGCTGTTGATATTCTTG), CDE représentant l'élément dépendant du cycle cellulaire *cell cycle dépendant element* (séquence nucléotidique TGGCGG), CHR représentant la région d'homologie de gène de cycle cellulaire *cell cycle gene homology region* (séquence nucléotidique GTTTGAA) et Inr représentant le site d'initiation (position +1) ainsi que les séquences voisines importantes pour l'initiation, et des variants et mutants fonctionnellement actifs du module promoteur étant également inclus.

3. Substance active selon la revendication 1, contenant une séquence d'activateur qui est régulée par des facteurs de transcription formés dans des cellules endothéliales ou dans de cellules gliales.

4. Substance active selon la revendication 2, contenant en tant que séquence d'activateur
- le promoteur de CMV, l'activateur de CMV ou le promoteur de SV40, ou
- la séquence de promoteur pour le transporteur de glucose 1 endothélial, spécifique du cerveau, pour l'endogline, pour le récepteur de VEGF-1, 2, la tyrosine kinase de récepteur til-1 ou til-2, le récepteur B61, le ligand B61 pour endothéline, en particulier endothéline B ou endothéline 1, pour des récepteurs d'endothéline, pour le récepteur de mannose-6-phosphate, IL-1α ou IL-1β, le récepteur d'IL-1, VCAM-1, le facteur von Willebrand, ou
- des sites de liaison oligomérisés pour des facteurs de transcription qui sont actifs préférentiellement ou sélectivement dans des cellules endothéliales, comme par exemple GATA-2 avec son site de liaison 5'-TTATCT-3', ou
- des séquences de promoteur pour la périaxine spécifique de cellules de Schwann, pour la glutamine synthétase, la protéine spécifique de cellules gliales, la protéine de cellule gliale S100b, l'interleukine 6, les récepteurs de 5-hydroxytryptamine, TNFα, IL-10 ou des récepteurs de facteur de croissance ressemblant à l'insuline, ou
- la séquence de promoteur du VEGF, la séquence d'activateur de VEGF ou la séquence d'ADN de v-Scr ou la séquence d'ADN de c-Src, qui régulent le gène de VEGF.

5. Substance active selon les revendications 1 à 3, dans laquelle la séquence d'ADN pour le facteur neurospécifique code
- pour un facteur de croissance neuronale, en particulier FGF, NGF, BDNF, NT-3, NT-4 ou CNTF, ou
- TGFβ, un récepteur soluble de TNF, IL-10, un récepteur soluble d'IL-1 ou un récepteur soluble d'IL-6 ou les antagonistes du récepteur d'IL-1, ou une protéine de fusion d'un cytokine (par exemple TGFβ, IL-10 ou un antagoniste de récepteur d'IL-1) ou d'un récepteur soluble de cytokine (par exemple le récepteur de TNF, le récepteur d'IL-6 ou le récepteur d'IL-1) avec le fragment Fc de l'immunoglobuline humaine, ou
- pour une tyrosine hydroxylase ou une dopa-décarboxylase.

6. Substance active selon les revendications 1 à 5, qui contient les séquences d'ADN de deux facteurs neuro-spécifiques identiques ou de deux facteurs neuro-spécifiques différents, les deux séquences d'ADN étant reliées l'une à l'autre par une séquence d'ADN codant pour le site d'entrée interne du ribosome.

7. Substance active selon les revendications 1 à 6, insérée dans un vecteur.

8. Substance active selon la revendication 7, dans laquelle le vecteur est un virus.

9. Substance active selon la revendication 8, dans laquelle le virus représente un rétrovirus, un adénovirus, un virus associé à un adénovirus, *l'Herpes simplex* virus ou le virus de la vaccine.

10. Substance active selon les revendications 1 à 6, insérée dans un plasmide.

11. Substance active selon les revendications 7 à 10, préparée dans un système de dispersion colloïdale.

12. Substance active selon la revendication 11, dans laquelle le système de dispersion colloïdale consiste en liposomes.

13. Substance active selon la revendication 12, dans laquelle le système de dispersion colloïdale consiste en ligands-polylysine.

14. Substance active selon l'une des revendications 7 à 13, qui est complétée par un ligand qui se lie à des structures membranaires de cellules endothéliales ou de cellules gliales.

15. Substance active selon la revendication 14, dans laquelle le ligand
- est un anticorps monoclonal ou polyclonal ou un fragment d'anticorps de celui-ci, qui se lie par ses domaines variables à des structures membranaires de cellules endothéliales ou de cellules gliales, ou
- est une substance portant un résidu mannose en bout de chaîne, une cytokine ou un facteur de croissance ou un fragment ou une séquence partielle de ceux-ci, qui se lie à des récepteurs sur des cellules endothéliales ou des cellules gliales.

16. Substance active selon la revendication 15, dans laquelle les structures membranaires représentent
- un récepteur pour une cytokine ou un facteur de croissance tel qu'IL-1, FGF, PDGF, VEGF (en particulier FIT-1 et KDR), TGFβ, l'insuline ou le facteur de croissance ressemblant à l'insuline (ILGF), ou
- une molécule d'adhésion telle que SLeX, LFA-1, MAC-1, LECAM-1 ou VLA-4, ou
- le récepteur du mannose-6-phosphate.

17. Substance active selon les revendications 1 à 16, dans une composition médicamenteuse destinée à l'injection intraveineuse ou intra-artérielle, à l'application locale à l'endroit d'un traumatisme ou à l'injection dans la cavité du système nerveux central.
